Europäisches Patentamt

European Patent Office

Office européen des brevets

(11) Publication number: **0 432 629 A1**

## EUROPEAN PATENT APPLICATION

(21) Application number: 90123350.2

(51) Int. Cl.5: **C07K 3/18**

(22) Date of filing: 05.12.90

(30) Priority: 15.12.89 JP 323773/89

(43) Date of publication of application:
19.06.91 Bulletin 91/25

(84) Designated Contracting States:
CH DE FR GB LI SE

(71) Applicant: Tosoh Corporation
4560, Kaisei-cho
Shinnanyo-shi, Yamaguchi-ken 746(JP)

(72) Inventor: Nakamura, Koji
3079-3, Oaza-Tonda
Shinnanyo-shi, Yamaguchi-ken(JP)

(74) Representative: Wächtershäuser, Günter, Dr.
Tal 29
W-8000 München 2(DE)

(54) Method for binding immunoglobulin G to protein A.

(57) A method for binding immunoglobulin G (IgG) to protein A immobilized on an insoluble matrix, wherein a buffer solution having a pH of at least 7 containing at least 0.2 M of a citrate or tartarate is used.

EP 0 432 629 A1

# METHOD FOR BINDING IMMUNOGLOBULIN G TO PROTEIN A

The present invention relates to a method for binding immunoglobulin G (hereinafter referred to simply as IgG) and more particularly, to a method of binding IgG to protein A which is immobilized on an insoluble matrix.

Protein A is capable of binding to the Fc portion of IgG and has been employed as a means for separating and purifying IgG, as immobilized on an insoluble matrix.

The binding of protein A and IgG was first discovered by Kronvall et al (Journal of Immunology, 105, 1116 (1970)).

Subsequently, Mackenzie et al attempted to improve the separation of IgG using a protein A-Sepharose 4B column. However, the amounts of mouse IgG 1 and IgG 2 thereby bound were small, and the reproducibility was also poor (Journal of Immunology, 120, 1493 (1978)). Bywater et al studied purification of IgG employing 16 different types of elution buffers, but were unable to improve over the conventional methods (Journal of Immunological Methods, 64, 1 (1983)). On the other hand, Hector et al improved the binding of IgG to protein A immobilized on an insoluble matrix by using a buffer solution having a pH of from 8.5 to 9.5 containing from 1 to 4 M of an inorganic salt (US patent 4,704,366). However, this method had drawbacks such that the concentration of the salt was so high that proteins precipitated, the viscosity of the solution was also high, and particularly in the case of a salt containing halogen ions, corrosion was likely to result at the stainless steel portions of the apparatus for separation and purification.

It is an object of the present invention to overcome such drawbacks of the conventional methods and to present a method provided with various properties required for the binding of IgG to protein A immobilized on an insoluble matrix.

The present invention has been made to solve the problems of the prior art.

The present inventors have solved such problems by conducting the binding of IgG to protein A immobilized on an insoluble matrix in a solution having certain specific liquid properties.

Namely, the present invention provides a method for binding immunoglobulin G (IgG) to protein A immobilized on an insoluble matrix, wherein a buffer solution having a pH of at least 7 containing at least 0.2 M of a citrate or tartarate is used.

Now, the present invention will be described in detail with reference to the preferred embodiments.

As the citrate or tartarate to be used in the present invention, any citrate or tartarate may be employed so long as it is soluble in water. The concentration of the salt is at least 0.2 M, preferably from 0.4 to 2 M.

The salt employed to obtain a buffer solution having a pH of at least 7, may be any salt so long as it has a buffering ability to bring the pH to from 7 to 9.5.

The insoluble matrix may be any matrix such as polyacrylamide, agarose, cellulose, polyhydroxyalkyl methacrylate or silica.

Now, the present invention will be described in further detail with reference to Examples. However, it should be understood that the present invention is by no means restricted to such specific Examples.

Example 1

1 m$\ell$ of protein A-Sepharose 4B (trade name of Pharmacia Company) was packed into an open column (inner diameter: 0.8 cm, length: 10 cm) and equilibrated with 5 m$\ell$ of buffer solution A (0.1 M glycine + 0.4 M sodium citrate (pH 9.0)). 0.5 m$\ell$ of mouse ascites fluid (containing 7 mg of monoclonal antibody IgG to human albumin) (Cedarlane Company) was diluted with 0.5 m$\ell$ of buffer solution A and added to the column to let IgG be bound to protein A.

The column was washed with 10 m$\ell$ of buffer solution A. Then, 3 m$\ell$ of 0.1 M glycine-HC$\ell$ (pH 2.8) was passed therethrough to desorb and recover IgG, whereby 96% of IgG was recovered.

Example 2

1 m$\ell$ of protein A-Sepharose 4B was packed into an open column (inner diameter: 0.8 cm, length: 1 cm) and equilibrated with 5 m$\ell$ of buffer solution B (0.1 M glycine + 0.4 M sodium tartarate (pH 9.0)), then, 0.5 m$\ell$ of the same mouse ascites fluid as identified in Example 1 was diluted with 0.5 m$\ell$ of buffer solution B and added to the column to let IgG be bound to protein A. The column was washed with 10 m$\ell$ of buffer solution B. Then, IgG was recovered with 3 m$\ell$ of 0.1 M glycine-HC$\ell$ (pH 2.8), whereby 98% of IgG was recovered.

Comparative Example

1 m$\ell$ of protein A-Sepharose 4B was packed into an open column (inner diameter: 0.8 cm, length: 10 cm) and equilibrated with 5 m$\ell$ of buffer solution C (0.1 M glycine (pH 9.0)). 0.5 m$\ell$ of the same mouse ascites fluid as identified in Example 1 was diluted with 0.5 m$\ell$ of buffer solution C and added to the column. The column was washed with 10 m$\ell$ of buffer solution C. Then, IgG was recovered with 3 mℓ of 0.1 M glycine-HCℓ (pH 2.8). The recovery rate was 20%, and the rest i.e. 80% was not adsorbed by the protein A column.

## Claims

1. A method for binding immunoglobulin G (IgG) to protein A immobilized on an insoluble matrix, wherein a buffer solution having a pH of at least 7 containing at least 0.2 M of a citrate or tartarate is used.

2. The method according to Claim 1, wherein the buffer solution contains from 0.4 to 2 M of the citrate or tartarate.

3. The method according to Claim 1, wherein the buffer solution has a pH of from 7 to 9.5.

4. The method according to Claim 1, wherein the insoluble matrix is polyacrylamide, agarose, cellulose, polyhydroxyalkyl methacrylate or silica.

European
Patent Office

**EUROPEAN SEARCH
REPORT**

Application Number

**EP 90 12 3350**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | GB-A-2 160 530   (BIO-RAD LABORATORIES INC.) <br> * claims * <br> − − − | 1-4 | C 07 K <br> 3 18 |
| Y | EP-A-0 304 161   (TOSOH CORPORATION) <br> * column 2, lines 29 - 34 * * claims * <br> − − − | 1-4 | |
| A | EP-A-0 323 027   (BIOPROBE INTERNATIONAL INC.) <br> * claims * <br> − − − | 1-4 | |
| P,X | EP-A-0 391 526   (BIOPROBE INTERNATIONAL INC.) <br> * page 4, line 46 - page 5, line 40 * <br> − − − − − | 1-4 | |

TECHNICAL FIELDS
SEARCHED (Int. Cl.5)

C 07 K
C 12 P
A 61 K

The present search report has been drawn up for all claims

| Place of search | Date of completion of search | Examiner |
|---|---|---|
| The Hague | 25 March 91 | NOOIJ F.J.M. |